# FASCICULE DE BREVET EUROPEEN

(11) **EP 0 731 912 B1**
(45) Date de publication et mention de la délivrance du brevet: **04.12.2002**
(21) Numéro de dépôt: 95933454.1
(22) Date de dépôt: 29.09.1995
(51) Int. Cl.: G01N 33/28, G01N 33/24, G01N 25/00, G01N 7/00

(54) **DISPOSITIF POUR DETERMINER SUR UN SITE D'EXPLOITATION DES CARACTERISTIQUES D'ECHANTILLONS DE FLUIDES PETROLIERS PAR EXEMPLE**
VORRICHTUNG ZUR FESTSTELLUNG AN DEM BORORT DER EIGENSCHAFTEN VON PROBEN VON ZUM BEISPIEL ERDÖLFLÜSSIGKEITEN
DEVICE FOR DERTERMINING ON AN ACTIVE SITE SAMPLE CHARACTERISTICS OF FOR EXAMPLE PETROLEUM FLUIDS

(30) Priorité: 30.09.1994 FR 9411817
(43) Date de publication de la demande: 18.09.1996
(73) Titulaire: INSTITUT FRANCAIS DU PETROLE, 92500 Rueil Malmaison (FR); SOCIETE ROP, 95101 Argenteuil Cédex (FR)
(72) Inventeur: MORACCHINI, Gérard Les Hauts-de-Maugarny, F-95580 Andilly (FR); BEHAR, Emmanuel, F-95000 Jouy-le-Moutier (FR); SANCHEZ, José, F-95270 Viarmes (FR)
(86) Numéro de dépôt international: FR9501263
(87) Numéro de publication internationale: WO96010745

(56) Documents cités:
- EP-A- 0 180 844
- EP-A- 0 473 472
- FR-A- 2 593 921

## Description

La présente invention concerne un dispositif pour déterminer sur un site d'exploitation, des caractéristiques d'échantillons de fluides extraits du sous-sol, et notamment de zones pétrolifères et plus particulièrement un dispositif de chantier compact pouvant être facilement transporté jusque sur des sites de production.

Le dispositif selon l'invention permet de réaliser sur site des mesures thermodynamiques étendues en déchargeant si nécessaire, les opérateurs de la conduite de nombreuses séquences opératoires. Il permet aussi de valider des échantillons, pour s'assurer qu'ils sont bien représentatifs des prélèvements qui ont été effectués, avant leur envoi à un laboratoire central pour des analyses plus complètes.

Par ces mesures et analyses préalables menées sur site avec des échantillons de très petits volumes, on évite les solutions classiques longues et coûteuses d'envoi systématique à un laboratoire éloigné d'échantillons à réitérer parfois quand il s'avère qu'ils ne sont pas valides et représentatifs. L'utilisation du dispositif selon l'invention procure de ce fait un gain de temps et des économies substantielles.

Le dispositif selon l'invention permet par exemple dans le cas d'échantillons pétroliers de type huile, de mesurer leur G.O.R (gas-oil ratio), le "point de bulle" et la masse spécifique de l'huile, et dans le cas d'échantillons de type gaz, de mesurer le "point de rosée" de la phase gazeuse, d'établir la courbe des dépôts liquides, ou encore de faire une analyse qualitative du mélange gazeux.

Comme on le sait, il est d'usage de conduire une étude des propriétés thermodynamiques d'échantillons extraits de puits forés en vue de la mise en production de gisements souterrains d'effluents tels que des effluents pétroliers. Par une bonne connaissance de ces propriétés thermodynamiques, il est possible d'évaluer les réserves enfouies, d'optimiser l'installation de mise en production ou bien encore de spécifier les méthodes opératoires. Les propriétés thermodynamiques sont calculées en utilisant des modèles compositionnels dans lesquels on intègre des données obtenues par l'analyse des échantillons.

Une méthode d'analyse du type dit "PVT" permet par exemple de mesurer le comportement d'échantillons dont on fait varier certains paramètres tels que la pression et la température par exemple depuis des valeurs reproduisant celles qui existent à leur profondeur d'enfouissement jusqu'à celles régnant en surface. Par des méthodes d'analyse de ce type, on arrive à mesurer des propriétés importantes telles que le "point bulle" indiquant l'apparition d'une phase gazeuse dans l'échantillon testé, le coefficient de compressibilité, la viscosité, la masse volumique, le G.O.R.etc, comme il est bien connu des spécialistes.

Par le brevet FR 2 666 415 du demandeur, on connait un dispositif pour faire au laboratoire ou sur un chantier, des mesures thermodynamiques sur des échantillons de faible volume. Ce dispositif comporte une enceinte thermostatée, une structure de support avec deux plateaux de part et d'autre d'un bloc transparent en saphir par exemple et deux chambres cylindriques de faible section délimitées par deux pistons coulissants déplacés dans les deux chambres par des moyens d'entraînement mécaniques: tiges filetées mises en rotation par des arbres couplés avec des moyens moteurs. Des joints d'étanchéité sont placés dans des rainures ménagées dans la paroi des deux chambres, autour des deux pistons. La première chambre est en partie visible au travers du bloc transparent, et elle est terminée par un biseau en haut duquel débouche un canal communiquant par l'intermédiaire d'une vanne avec la deuxième chambre. Un capillaire relie les deux chambres et permet de faire des mesures de viscosité sur les échantillons. Du fait de sa compacité, ce dispositif antérieur est facilement transportable jusqu'aux sites de prélèvement des échantillons.

De tels dispositifs sont aussi connus par les brevets EP 0 180 844 A et FR 2 593 921 A.

Le dispositif selon l'invention permet de conduire directement sur un site d'exploitation, des caractéristiques d'échantillons de fluides extraits du sous-sol, et notamment de zones pétrolifères. Il comporte dans une enceinte thermostatée, un corps comprenant une première chambre et une deuxième chambre disposée au-dessus de la première, la première chambre au moins comportant une extrémité de forme pointue, les volumes de ces deux chambres pouvant varier par déplacement d'éléments mobiles dans deux cylindres, des moyens de déplacement des deux éléments mobiles, des moyens de transfert de fluides dans ou hors des chambres, et des moyens de communication contrôlée entre les deux chambres.

Le dispositif est caractérisé en ce que le corps comporte deux cavités radiales coaxiales débouchant dans cette première chambre dans sa partie pointue, pour un ensemble optique de visualisation constitué de deux éléments optiques insérés de façon étanche respectivement dans les deux cavités, comportant chacun un manchon rigide, un bloc cylindrique en un matériau transparent tel que du saphir disposé dans l'axe du manchon rigide et des moyens de fixation d'un embout de fibre optique relié à un élément photo-émetteur ou photo-récepteur, pour former l'image de l'extrémité de la première chambre.

L'utilisation de ces blocs de visualisation à fibres optiques permet par des moyens de détection du ménisque entre la phase liquide et la phase gazeuse de l'échantillon, d'automatiser les opérations de séparation des phases avec détermination du G.O.R..

Suivant un premier mode de mise en oeuvre, les deux chambres sont délimitées respectivement par deux tiges formant pistons pourvues chacune de joints d'étanchéité, le dispositif comportant des moyens moteurs pour contrôler le déplacement des deux tiges, et un gazomètre disposé dans l'enceinte thermostatée, ce qui contribue à raccourcir les circuits d'interconnexion et à diminuer les risques de condensation, causes habituelles d'erreurs de mesure.

Suivant un premier mode de mise en oeuvre, la première chambre est délimitée par une tige formant piston pourvue de joints d'étanchéité, et la deuxième chambre est constituée du volume intérieur d'un gazomètre à piston mobile surmontant la première chambre.

Ce mode de mise en oeuvre où la cellule et le gazomètre sont intégrés, conduit à un appareil plus léger et donc plus facilement déplaçable sur les sites d'exploitation avec un circuit de connexion très court entre la cellule et le gazomètre, ce qui est favorable à la précision des mesures.

Suivant un mode de réalisation, le corps est fixé à l'intérieur de l'enceinte thermostatée, les deux éléments mobiles ( et au moins la tige dans la première chambre basse dans le cas où elle est surmontée d'un gazomètre) sont solidaires respectivement de deux étriers déplaçables chacun parallèlement aux deux pistons par la mise en rotation respectivement de tiges filetées et des moyens moteurs (tels que des moteurs pas-à-pas ou des moteurs synchrones) pour faire tourner séparément ou de façon sychrone les tiges associées à chaque étrier.

Le piston du gazomètre coulisse par exemple dans un cylindre et il est pourvu d'une tige reliée à des moyens moteurs adaptés à limiter la pression dans le cylindre à une valeur de consigne, de façon à pouvoir mesurer à cette pression le volume de gaz dégagé par la détente du mélange.

Le dispositif comprend de préférence un ensemble de contrôle des moyens moteurs et de l'ensemble optique de visualisation, comportant par exemple un module de commande des moyens moteurs pour l'entraînement des tiges filetée et un circuit de mesure par codage optique pour détecter les mouvements transmis aux tiges filetées et les transformer en mesures des variations de volume, et éventuellement un module pour commander l'éclairement de la première chambre à travers du premier élément optique, et former l'image d'une interface entre des phases au travers du deuxième élément optique.

Le dispositif peut comporter en outre un vibrateur pour agiter les fluides dans la première chambre, l'ensemble de contrôle comportant un circuit produisant le signal à appliquer au vibrateur.

Le dispositif comporte avantageusement un micro-ordinateur programmé pour réaliser des procédures automatiques de mesure.

Suivant un mode de réalisation, le dispositif comporte des moyens de mesure de la pression au moins dans la première chambre, l'ensemble de contrôle comportant un circuit de mémorisation des facteurs d'étalonnage de ces moyens de mesure, en fonction d'une température de consigne associé à un élément de sélection des facteurs d'étalonnage à choisir en fonction de cette température de consigne assignée, pour rendre la réponse du moyen de mesure indépendante de celle-ci.

Les moyens de mesure de la pression comprennent au moins un capteur de pression à membrane disposé dans une cavité dans la paroi de la première chambre ménagée dans la paroi de la première chambre avec la membrane affleurant la surface intérieure de cette chambre de façon à limiter les volumes morts.

Les joints d'étanchéité étant mobiles avec les pistons, les volumes morts entre les deux cylindres et les pistons correspondants restent sensiblement constants quel que soit leur degré d'enfoncement dans leurs chambres respectives.

Suivant un mode de réalisation, le corps est fixé à l'intérieur de l'enceinte thermostatée, les deux pistons sont solidaires respectivement de deux étriers déplaçables chacun parallèlement aux deux pistons par la mise en rotation respectivement de tiges filetées et des moyens moteurs (des moteurs pas-à-pas par exemple) pour faire tourner séparément les tiges associées à chaque étrier.

Le dispositif comporte de préférence un ensemble de contrôle des moyens moteurs et de l'ensemble optique.

Cet ensemble de contrôle comporte par exemple un module de commande des moyens moteurs d'entrainement des tiges filetées et un circuit de mesure par codage optique pour détecter les mouvements transmis aux tiges filetées et les transformer en mesures des variations de volume concomitantes des deux chambres. Il peut comporter aussi un module pour commander l'éclairement de la première chambre à travers le premier élément optique et former l'image d'une interface entre des phases fluides de l'échantillon au travers du deuxième élément optique.

Le dispositif peut comporter aussi un vibrateur pour agiter les fluides dans la première chambre, et l'ensemble de contrôle, aussi, un circuit produisant le signal à appliquer au vibrateur.

Le dispositif comporte encore par exemple un moyen de mesure de la pression dans la première chambre, l'ensemble de contrôle comportant dans ce cas un circuit de mémorisation des facteurs d'étalonnage de ce moyen de mesure en fonction d'une température de consigne, associé à un élément de sélection des facteurs d'étalonnage à choisir en fonction de cette température de consigne assignée, pour rendre la réponse du moyen de mesure indépendante de celle-ci.

De préférence, le dispositif comporte un micro-ordinateur programmé pour réaliser des procédures automatiques de mesure.

D'autres caractéristiques et avantages du dispositif selon l'invention, apparaîtront à la lecture de la description ci-après d'un mode de réalisation décrit à titre d'exemple non limitatif, en se référant aux dessins annexés où :
- la Fig. 1 montre schématiquement une première vue en coupe du dispositif dans son enceinte thermostatée;
- la Fig.2 montre schématiquement une autre vue en coupe du dispositif;
- la Fig.3 montre une vue en coupe schématique des moyens d'entraînement des tiges filetées en rotation;
- la Fig.4 montre schématiquement le dispositif associé à des modules de contrôle;
- la Fig.5 montre schématiquement un premier mode de mise en oeuvre du dispositif convenant pour des mesures thermodynamiques sur des échantillons de fluides;
- la Fig.6 est un schéma fonctionnel de l'ensemble de contrôle associé à son micro-ordinateur de commande et
- la Fig.7 montre schématiquement un deuxième mode de mise en oeuvre du dispositif convenant pour valider des échantillons de fluides.

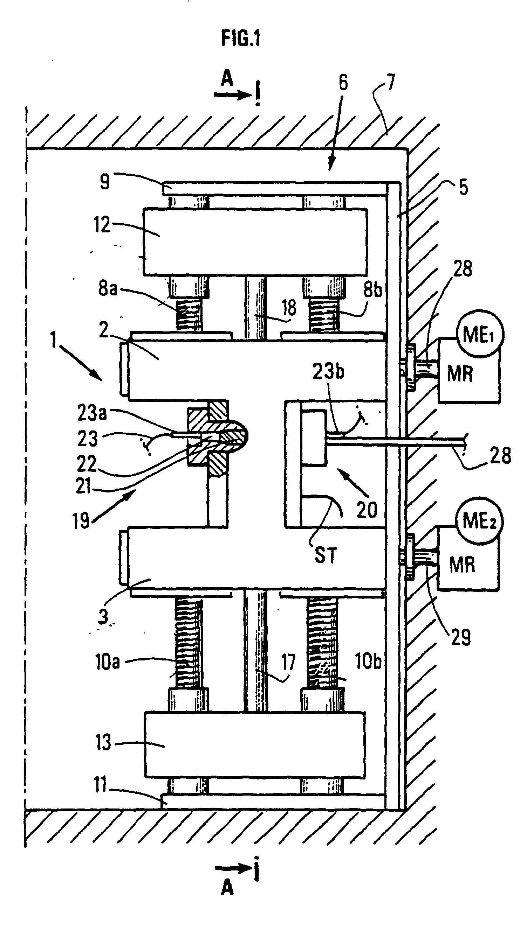

Le dispositif comporte (Fig.1) un corps rigide 1 constitué de deux plateaux 2, 3 séparés par un bloc cylindrique 4. Les deux plateaux du corps 1 sont fixés à un montant vertical 5 d'une cadre de support rigide 6. Le corps 1 est placé dans une enceinte thermostatée 7. Deux tiges filetées 8a, 8b latéralement écartées l'une de l'autre, et libres de tourner sur elles-mêmes, sont disposées entre un montant horizontal supérieur 9 du cadre 6 et le plateau supérieur 2. Deux tiges analogues 10a, 10b, alignées respectivement avec les tiges 8a, 8b, sont disposées entre le plateau inférieur 3 du corps et un autre montant horizontal inférieur 11 du cadre 6.

Deux étriers 12,13 sont disposés de part et d'autre du corps 1. Ils comportent des alésages filetés adaptés respectivement aux tiges filetées 8, 10. La translation verticale des étriers 12, 13, est obtenue par la mise en rotation respectivement de ces tiges filetées 8, 10.

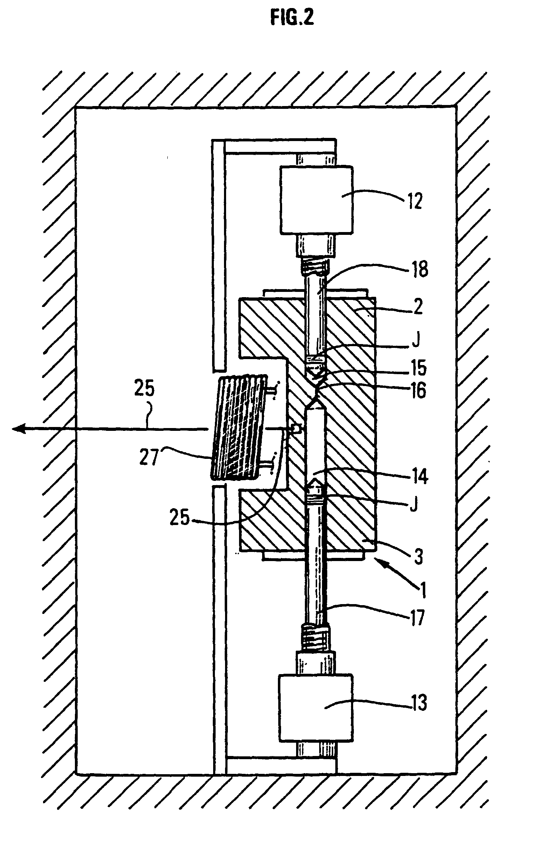

Deux chambres cylindriques coaxiales 14, 15 (Fig.2 à 5) avec un fond ou extrémité conique, sont ménagées au travers du corps 1. Elles communiquent l'une avec l'autre à leurs pointes par un canal fin 16 commandé par une vanne V5. Dans ces deux chambres coulissent deux pistons 17, 18 pourvus chacun d'une extrémité conique adaptée à la forme des deux chambres. Les deux pistons sont respectivement solidaires des deux étriers mobiles 12, 13. Chacun est pourvu au voisinage de sa pointe d'un joint d'étanchéité J. Le volume mort périphérique entre chaque pointe de piston et le joint J correspondant est ainsi constant quel que soit leur degré enfoncement à l'intérieur de leurs chambres respectives.

Le dispositif comporte un ensemble optique de visualisation constitué (Fig.1) de deux éléments optiques 19, 20. Deux cavités radiales coaxiales débouchant dans la chambre basse 14 au voisinage de son sommet, sont ménagées au travers du corps 1, respectivement pour les deux éléments optiques 19, 20. Chacun d'eux est constitué d'une monture métallique avec un alésage central pour un bloc transparent 21, en saphir par exemple. Chaque bloc est prolongé extérieurement par un embout 22 de fibre optique. Une des fibres optiques 23a communique avec une source de lumière telle qu'une diode photo-émettrice par exemple, la fibre optique 23b de l'élément optique opposé 20, avec un élément photo-récepteur permettant de former l'image du bec conique de la chambre basse 14.

Avec la conformation particulière à bec conique donnée à la zone de prélèvement en haut de la première chambre, avec le bloc de saphir 22 et le système d'éclairement, on obtient une très grande précision dans toutes les opérations que l'on peut mener sur les fluides dans la première chambre. C'est vrai en particulier, quand on doit prélever avec précision toute la phase gazeuse d'une substance diphasique sous pression dans cette première chambre basse 14 pour l'introduire dans la deuxième chambre 15.

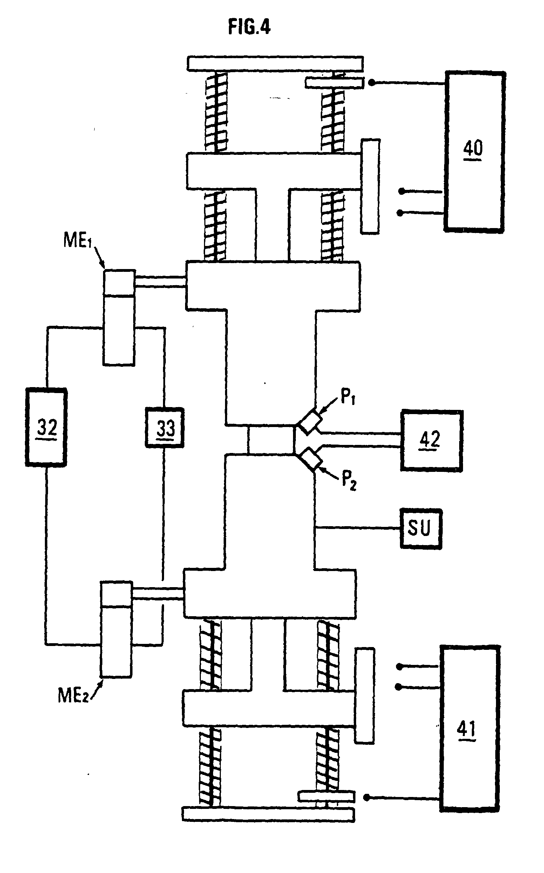

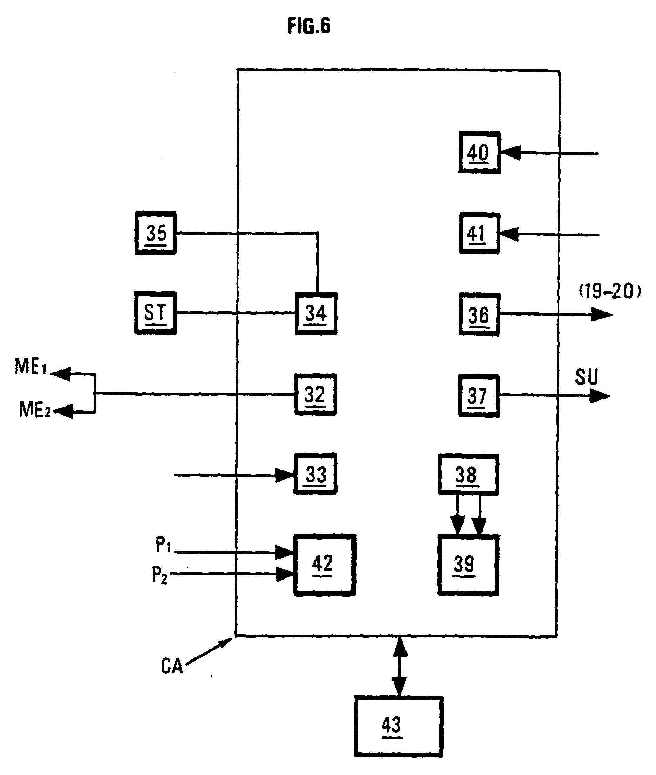

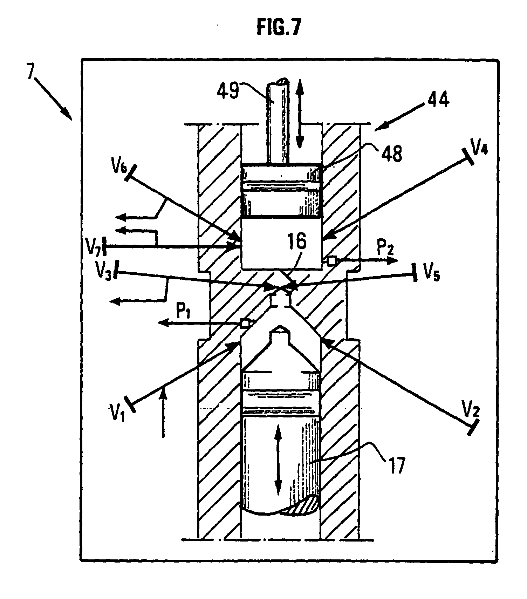

Le corps comporte une cavité ouvrant sur la chambre inférieure 14 vers le haut de celle-ci, pour un capteur de pression P1. De préférence, on utilise au moins dans la première chambre 14, un capteur à membrane affleurante P1 (cf. Fig.7), de façon à minimiser les volumes morts susceptibles d'altérer la précision des mesures. Des conducteurs 25 relient le capteur P1 à un ensemble de contrôle extérieur à l'enceinte thermostatée 7 qui sera décrit en relation avec les Fig.4 et 6.

En outre, une sonde de température ST est placée dans le corps 1 pour mesurer la température de l'échantillon de substance.

Au plateau inférieur 3, est fixé une céramique piézo-électrique (non représentée) associée à un générateur d'ultrasons SU (Fig.4) pour agiter l'échantillon de substance introduit dans la première chambre 14.

Les deux chambres 14, 15 communiquent respectivement par l'intermédiaire de vannes V2, V4 avec un serpentin capillaire 27 (Fig.5), permettant de faire des transferts de fluide de l'une à l'autre et ainsi de faire des mesures de viscosité. Une vanne V3 communiquant avec le canal fin 6 entre les deux chambres 14, 15, permet de purger la chambre inférieure 14. Une vanne V1 commande un accès à la chambre basse 14 pour l'introduction des échantillons fluides à analyser.

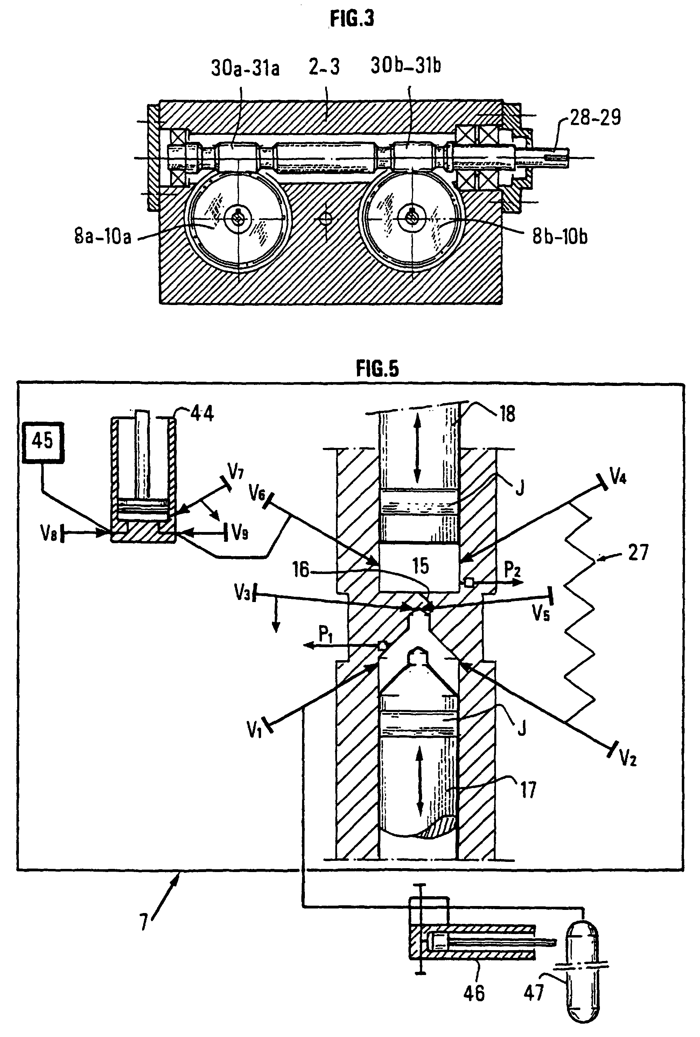

A chaque paire de tiges filetées 8a, 8b et 10a, 10b, est associé (Fig.3) respectivement un arbre d'entraînement 28, 29 disposé dans un plan perpendiculaire aux axes des tiges filetées. Chaque arbre est assujetti à l'un des plateaux 2, 3, mais il peut tourner sur lui-même. Chacun d'eux porte deux engrenages 30a, 31a, d'une part et 30b, 31b d'autre part, venant s'engrener sur les filetages des tiges correspondantes 8, 10. Les sorties des deux arbres 28, 29 sont couplées chacune avec un moteur d'entraînement pas-à-pas ME1, ME2 (Fig.1) dans un boitier adjacent à l'enceinte thermostatée.

Le dispositif comporte également un ensemble de contrôle CA (Fig.6) comprenant :
- un module 32 de commande des deux moteurs d'entrainement ME1, ME2 et un circuit 33 de mesure par codage optique pour détecter les mouvements transmis aux arbres d'entrainement 28, 29 des deux moteurs ME1, ME2 et les transformer en mesures des variations de volume concomitantes de la première et de la deuxième chambre 14, 15;
- un circuit 34 pour réguler la température. Ce circuit reçoit les données de température de la sonde ST. Il agit sur des éléments de chauffage 35 de l'enceinte thermostatée et il est adapté à limiter automatiquement la température maximale qui y règne;
- un module 36 pour commander l'éclairement de la chambre basse 14 à travers du hublot 19 et recevoir l'image de l'interface entre les phases reçue au travers du hublot 20;
- un circuit 37 produisant le signal à appliquer au générateur d'ultrasons SU;
- un circuit 38 de mémorisation des facteurs d'étalonnage du capteur de pression 26 en fonction de la température de consigne indiquée par la sonde de température ST, associé à un élément de sélection 39 des facteurs d'étalonnage à choisir en fonction de la température de consigne assignée, pour rendre la réponse du capteur 31 indépendante de celle-ci. L'ensemble de contrôle comporte également des circuits 40, 41 de détection de la position des étriers avec des indicateurs de fin de course ainsi q'un circuit de détection 42 connecté aux capteurs de pression P1, P2 pour détecter les dépassements de pression dans les chambres au-delà d'une pression de consigne.

Un micro-ordinateur 43 est associé à l'ensemble de contrôle pour conduire de façon automatique les séquences de mesures.

La section des chambres 14, 15 est suffisamment faible pour que l'on puisse faire des essais avec de petits volumes de substance, de l'ordre de quelques cm3 et les moyens moteurs sont adaptés à porter la pression dans ces chambres à plusieurs centaines de bars, par déplacement des tiges 17, 18 formant pistons. Les petits volumes mis en jeu permettent de minimiser les problèmes de sécurité d'une part et aussi la durée des étapes préalables de chauffage, d'agitation.

L'échantillon de substance étant soumis aux conditions de température et de pression régnant dans la zone souterraine où il a été prélevé, on peut dans un premier temps abaisser sa pression par retrait du premier piston 17 suffisamment pour pouvoir mesurer par exemple sa compressibilité. En abaissant encore sa pression, on provoque sa vaporisation partielle. Par ouverture de la vanne V5, il est possible de transférer toute la phase gazeuse dans la deuxième chambre 15.

Suivant le mode de mise en oeuvre de la Fig. 5, on associe le dispositif à un gazomètre 44 que l'on dispose dans l'enceinte climatique 7 à proximité du corps 1 de la cellule. Une entrée du gazomètre commandée par une vanne V9, est reliée à la deuxième chambre 15 par la vanne V6.

Par une vanne V7, le gazomètre peut être connecté avec une pompe à vide (non représentée). Avec ce gazomètre 44, on fait des mesures du volume du gaz prélevé dans la deuxième chambre après détente jusqu'à la pression atmosphérique. Cette proximité de la chambre 15 et du gazomètre 44 et leur maintien à la même température, qui évite toute condensation, permet de faire des mesures plus précises.

Le gazomètre est couplé par exemple avec un appareil de chromatographie 45 par l'intermédiaire d'une vanne V8. Par la vanne V1, la première chambre peut être couplée avec une micro-cellule de prélèvement d'un type connu 46 afin de mesurer la masse volumique de la substance étudiée.

Le transfert sous pression d'un échantillon dans la première chambre du dispositif peut aussi être fait directement depuis une bouteille 47 de transport. On peut aussi l'introduire dans la micro-cellule 46 à un stade intermédiaire, avant de le transférer dans la première chambre 17. La micro-cellule peut être pesée pour déterminer la masse introduite dans la cellule. On peut aussi déterminer la masse spécifique de la substance.

Le mode de réalisation de la Fig.5 convient particulièrement pour faire des études thermodynamiques sur des échantillons avec possibilité par exemple de mesurer la viscosité en couplant les deux chambres 15 et 17 par le serpentin 27. Dans le cas où l'on cherche seulement à valider un échantillon pour vérifier qu'il est bien représentatif des substances à analyser, on utilise le dispositif par exemple suivant le mode de réalisation de la Fig.7.

Sur la Fig.7, on voit que la deuxième chambre est constituée par le volume intérieur d'un gazomètre tel que 44. La tige 18 couplée avec les moyens moteurs (8, 12, ME1), est remplacée ici par un piston 48 coulissant dans un cylindre 49. La tige de ce piston est reliée à des moyens moteurs adaptés à maintenir la pression dans le cylindre 49 au plus égal à une valeur de référence, de façon à pouvoir mesurer à cette pression le volume de gaz dégagé par la détente du mélange. De la même façon, ce gazomètre est placé dans l'enceinte thermostatée 7 pour une meilleure précision des mesures.

Avec cet agencement où la cellule et le gazomètre sont intégrés, on réalise un appareil plus léger et donc plus facilement déplaçable sur les sites d'exploitation. En outre, le circuit de connexion de la cellule au gazomètre étant très court, ce qui élimine une cause connue d'imprécision des mesures.

Le micro-ordinateur 43 est adapté à réaliser certaines séquences d'acquisition et de conduite d'opérations.
- Il effectue par exemple un contrôle automatique de la pression lorsque, à une certaine température de consigne, on transfère en le détendant le gaz de la deuxième chambre dans le gazomètre 44, jusqu'à la pression atmosphérique, afin de déterminer son volume à des conditions standards (une correction est apportée pour donner le volume à 15°C). Il effectue aussi une programmation de la température de consigne à respecter. Sur commande de l'opérateur il procède à une acquisition des données mesurées de pression, de volume et de température (P, V, T).
- Le micro-ordinateur 43 peut conduire aussi une séquence de décompression automatique avec stabilisations successives à plusieurs paliers de pression. Dans ce cas, il scrute la pression et attend sa stabilisation pour procéder à l'acquisition des données P, V, T avant une nouvelle décompression.
- Sur commande de l'opérateur le micro-ordinateur 43 peut effectuer également toutes les mesures P, V, T nécessaires et les acquérir après validation, qu'il s'agisse de la pression à température de consigne, de la détermination des volumes des fluides contenus dans la première et dans la deuxième chambre à une température et une pression de consigne, du volume de la phase gazeuse obtenue à la température ambiante et la pression atmosphérique.
- Le micro-ordinateur 43 comporte des moyens de mémorisation pour enregistrer les données acquises.
- Par programmation, on peut faire effectuer au micro-ordinateur 43 un certain nombre d'opérations différentes suivant que l'on utilise le mode de réalisation de la Fig.5, pour déterminer des paramètres thermodynamiques de l'échantillon étudié et/ou des paramètres d'étalonnage, ou bien pour des opérations de validation d'échantillons suivant le mode de réalisation de la Fig.7.

## Revendications

1. Dispositif pour déterminer sur un site d'exploitation, des caractéristiques d'échantillons de fluides extraits du sous-sol, et notamment de zones pétrolifères, comportant, dans une enceinte thermostatée, un corps (1) comprenant une première chambre et une deuxième chambre disposée au-dessus de la première, la première chambre au moins comportant une extrémité de forme pointue, les volumes de ces deux chambres pouvant varier par déplacement d'éléments mobiles dans deux cylindres, des moyens de déplacement des deux éléments mobiles, des moyens de transfert de fluides dans ou hors des chambres, et des moyens de communication contrôlée entre les deux chambres, **caractérisé en ce que** le corps (1) comporte deux cavités radiales coaxiales débouchant dans cette première chambre (14) dans sa partie pointue, pour un ensemble optique de visualisation constitué de deux éléments optiques (19, 20) insérés de façon étanche respectivement dans les deux cavités, comportant chacun un manchon rigide, un bloc cylindrique (21) en un matériau transparent tel que du saphir disposé dans l'axe du manchon rigide et des moyens de fixation d'un embout de fibre optique (23a, 23b) relié à un élément photo-émetteur ou photo-récepteur, pour former l'image de l'extrémité de la première chambre.

2. Dispositif selon la revendication 1, **caractérisé en ce que** les deux chambres sont délimitées respectivement par deux tiges formant pistons pourvues chacune de joints d'étanchéité, le dispositif comportant des moyens moteurs pour contrôler le déplacement des deux tiges, et un gazomètre disposé dans l'enceinte thermostatée.

3. Dispositif selon la revendication 1, **caractérisé en ce que** la première chambre (14) est délimitée par une tige (17) formant piston pourvue de joints d'étanchéité (J), et la deuxième chambre est constituée du volume intérieur d'un gazomètre (44) à piston mobile (48) surmontant la première chambre.

4. Dispositif selon la revendication 2, **caractérisé en ce que** le corps est fixé à l'intérieur de l'enceinte thermostatée (7), les deux éléments mobiles (17, 18) sont solidaires respectivement de deux étriers (12, 13) déplaçables chacun parallèlement aux deux pistons par la mise en rotation respectivement de tiges filetées (8, 10) et des moyens moteurs (ME1, ME2) pour faire tourner séparément ou de façon sychrone les tiges associées à chaque étrier.

5. Dispositif selon la revendication 3, **caractérisé en ce que** le corps est fixé à l'intérieur de l'enceinte thermostatée (7), au moins un des éléments mobiles (17, 18) est solidaire d'un étrier (13) déplaçable parallèlement à l'élément mobile par la mise en rotation respectivement de tiges filetées (10) et des moyens moteurs (ME1, ME2) pour faire tourner séparément ou de façon sychrone les tiges associées à l'étrier.

6. Dispositif selon la revendication 3 ou 5, **caractérisé en ce que** le piston (48) du gazomètre (44) coulisse dans un cylindre et il est pourvu d'une tige (49) reliée à des moyens moteurs adaptés à limiter la pression dans le cylindre à une valeur de consigne, de façon à pouvoir mesurer à cette pression le volume de gaz dégagé par la détente du mélange.

7. Dispositif selon les revendications 4 ou 5, **caractérisé en ce qu'**il comporte des moteurs de type pas-à-pas ou des moteurs synchrones.

8. Dispositif selon l'une des revendications précédentes, **caractérisé en ce qu'**il comporte un ensemble (CA) de contrôle des moyens moteurs et de l'ensemble optique de visualisation.

9. Dispositif selon la revendication précédente, **caractérisé en ce que** l'ensemble de contrôle comporte un module (32) de commande des moyens moteurs (ME1, ME2) pour l'entrainement des tiges filetées (8, 10) et un circuit (33) de mesure par codage optique pour détecter les mouvements transmis aux tiges filetées et les transformer en mesures des variations de volume.

10. Dispositif selon la revendication 8, **caractérisé en ce que** l'ensemble de contrôle (CA) comporte un module (36) pour commander l'éclairement de la première chambre (14) à travers du premier élément optique (19), et former l'image d'une interface entre des phases au travers du deuxième élément optique (20).

11. Dispositif selon l'une des revendications 7 à 10, **caractérisé en ce qu'**il comporte un vibrateur (SU) pour agiter les fluides dans la première chambre (14), l'ensemble de contrôle (CA) comportant un circuit (37) produisant le signal à appliquer au vibrateur.

12. Dispositif selon l'une des revendications 7 à 11, **caractérisé en ce qu'**il comporte un moyen (25-26) de mesure de la pression dans la première chambre (14), l'ensemble de contrôle (CA) comportant un circuit (38) de mémorisation des facteurs d'étalonnage de ce moyen de mesure (25-26), en fonction d'une température de consigne associé à un élément (39) de sélection des facteurs d'étalonnage à choisir en fonction de cette température de consigne assignée, pour rendre la réponse du moyen de mesure (25-26) indépendante de celle-ci.

13. Dispositif selon l'une des revendications précédentes, **caractérisé en ce qu'**il comporte des moyens de mesure de la pression comprenant au moins un capteur de pression à membrane (PI) disposé dans une cavité ménagée dans la paroi de la première chambre avec la membrane affleurant la surface intérieure de cette chambre de façon à limiter les volumes morts.

14. Dispositif selon la revendication 2, **caractérisé en ce qu'**il comporte des moyens (27) de mesure de la viscosité connectés entre la première et la deuxième chambre (14, 15).

15. Dispositif selon l'une des revendications précédentes, **caractérisé en ce qu'**il comporte un micro-ordinateur (43) programmé pour réaliser des procédures automatiques de mesure.

## Patentansprüche

1. Vorrichtung zum Bestimmen der Charakteristika von unterirdisch geförderten Fluidproben und insbesondere von Erdölzonen an der Lagerstätte, welche Vorrichtung in einem thermostatischen Gefäß einen Körper (1), der eine erste Kammer und eine zweite auf der ersten angeordnete Kammer umfasst, wobei die erste Kammer wenigstens ein Ende mit spitzer Form umfasst, die Volumina dieser beiden Kammern durch Verschieben von beweglichen Elementen in zwei Zylindern variieren können, und Mittel zum Verschieben der beiden beweglichen Elemente, Mittel zum Fluidtransfer innerhalb oder außerhalb der Kammer und Mittel zum kontrollierten Kommunizieren zwischen den beiden Kammern umfasst, **dadurch gekennzeichnet, dass** der Körper (1) zwei radiale koaxiale Hohlräume, die in der ersten Kammer (14) in deren spitzem Ende münden, für eine optische Visualisierungseinheit, die aus optischen Elementen (19, 20) besteht, welche jeweils abdichtend in die beiden Hohlräume eingesetzt sind, und jedes eine steife Manschette, einen zylindrischen Block (21) aus transparentem Material wie Saphir, der in der Achse der steifen Manschette angeordnet ist, und Mittel zum Befestigen eines Ansatzstücks einer optischen Faser (23a, 23b) umfasst, das mit einem Photoemissionselement oder Photorezeptorelement verbunden ist, um das Bild des Endes der ersten Kammer zu ergeben.

2. Vorrichtung nach Anspruch 1, **dadurch gekennzeichnet, dass** die beiden Kammern jeweils durch zwei Stangen begrenzt sind, die Kolben bilden, welche jede als Dichtverbindungen vorgesehen sind, wobei die Vorrichtung Motormittel zur Steuerung der Verschiebung der beiden Stangen und ein Gasometer, das in dem thermostatischen Gefäß angeordnet ist, umfasst.

3. Vorrichtung nach Anspruch 1, **dadurch gekennzeichnet, dass** die erste Kammer (14) durch eine Stange (17) begrenzt ist, die einen Kolben bildet, der mit Dichtungsverbindungen (J) versehen ist, und die zweite durch das Innenvolumen eines Gasometers (44) mit beweglichen Kolben (48) gebildet ist, welches die erste Kammer übersteigt.

4. Vorrichtung nach Anspruch 2, **dadurch gekennzeichnet, dass** der Körper in dem thermostatischen Gefäß (7) befestigt ist, die beiden beweglichen Elemente (17, 18) mit zwei Spannern (12, 13) jeweils verbunden sind, die jeder parallel zu den beiden Kolben jeweils durch Rotation von Gewindestangen (8, 10) verschiebbar sind, und Motormittel (ME1, ME2), um unabhängig oder synchron die jedem Spanner zugeordneten Stangen drehen zu lassen.

5. Vorrichtung nach Anspruch 3, **dadurch gekennzeichnet, dass** der Körper in dem thermostatischen Gefäß (7) befestigt ist, wenigstens eines der beweglichen Elemente (17, 18) mit einem Spanner (13) verbunden ist, der parallel zu dem Element verschiebbar ist, das durch jeweiliges Rotieren der Gewindestangen (10) und den Motormitteln (ME1, ME2) parallel verschiebbar ist, um unabhängig oder synchron die dem Spanner zugeordneten Stangen drehen zu lassen.

6. Vorrichtung nach Anspruch 3 oder 5, **dadurch gekennzeichnet, dass** der Kolben (48) des Gasometers (44) in einen Zylinder stößt und eine Stange (49) vorgesehen ist, die mit Motormitteln verbunden ist, welche ausgelegt sind, um den Druck in dem Zylinder auf einen vorgeschriebenen Wert derart zu begrenzen, dass bei diesem Druck das durch Entspannung der Mischung freigesetzte Gasvolumen gemessen werden kann.

7. Vorrichtung nach einem der Ansprüche 4 oder 5, **dadurch gekennzeichnet, dass** sie Motoren vom schrittweisen Typ oder synchrone Motoren umfasst.

8. Vorrichtung nach einem der vorhergehenden Ansprüche, **dadurch gekennzeichnet, dass** sie eine Regeleinheit (CA) der Motormittel und der optischen Visualisierungseinheit umfasst.

9. Vorrichtung nach einem vorhergehenden Ansprüche, **dadurch gekennzeichnet, dass** die Regeleinheit ein Steuerungsmodul (32) der Motormittel (ME1, ME2) zum Antrieb der Gewindestangen (8, 10) und einen Messkreis (33) durch optische Kodierung umfasst, um die zu den Gewindestangen übertragenen Bewegungen zu detektieren und sie in Volumenänderungsmaße umzuwandeln.

10. Vorrichtung nach Anspruch 8, **dadurch gekennzeichnet, dass** die Regeleinheit (CA) ein Modul (36) zum Steuern der Beleuchtungsstärke der ersten Kammer (14) entlang des ersten optischen Elements (19) und Erzeugen eines Bildes einer Schnittstelle zwischen den Phasen entlang des zweiten optischen Elements (20) umfasst.

11. Vorrichtung nach einem der Ansprüche 7 bis 10, **dadurch gekennzeichnet, dass** sie einen Vibrator (SU) zum Schütteln der Fluide in der ersten Kammer (14) der Regeleinheit (CA) umfasst, welche einen Kreislauf (37) umfasst, der das auf den Vibrator anzuwendende Signal erzeugt.

12. Vorrichtung nach einem der Ansprüche 7 bis 11, **dadurch gekennzeichnet, dass** sie ein Mittel (25-26) zur Druckmessung in der ersten Kammer (14) umfasst, wobei die Regeleinheit (CA) einen Kreislauf (38) zur Speicherung von Eichfaktoren dieser Messmittel (25-26) in Abhängigkeit einer vorgeschriebenen, mit einem Element (39) zur Auswahl der Eichfaktoren verbundenen Temperatur umfasst, die in Abhängigkeit dieser zugeordneten und vorgeschriebenen Temperatur zu wählen sind, um die Antwort des Messmittels (25-26) unabhängig davon zu machen.

13. Vorrichtung nach einem der vorhergehenden Ansprüche, **dadurch gekennzeichnet, dass** sie Mittel zum Messen des Druckes umfasst, welche wenigstens einen Drucksensor mit Membran (P1) umfassen, der in einem Hohlraum angeordnet ist, der in der Wand der ersten Kammer mit der Membran ausgespart ist, die sich an die innere Oberfläche dieser Kammer anschließt, um die Totvolumen zu begrenzen.

14. Vorrichtung nach Anspruch 2, **dadurch gekennzeichnet, dass** sie Mittel (27) zum Messen der Viskosität umfasst, welche zwischen der ersten und der zweiten Kammer (15, 14) verbunden sind.

15. Vorrichtung nach einem der vorhergehenden Ansprüche, **dadurch gekennzeichnet, dass** sie einen Mikrocomputer (43) umfasst, der programmiert ist, um automatische Messverfahren durchzuführen.

## Claims

1. A device for determining, on a production site, characteristics of fluid samples extracted from the subsoil, notably from petroliferous areas, comprising, in a thermostat-controlled enclosure, a body (1) comprising a first chamber and a second chamber arranged above the first one, the first chamber at least comprising a pointed end, and the volumes of these two chambers can be varied by shifting mobile elements in two cylinders, means for shifting the two mobile elements, means for transferring fluids into or out of the chambers, and controlled communication means between the two chambers, **characterized in that** body (1) comprises two coaxial radial cavities opening into this first chamber (14) in the pointed part thereof, for an optical display assembly consisting of two optical elements (19, 20) tightly inserted respectively in the two cavities, comprising each a rigid sleeve, a cylindrical block (21) made of a transparent material such as sapphire arranged in line with the rigid sleeve and means for fastening an end of an optical fiber (23a, 23b) connected to a photoemission or photoreception element, for forming the image of the end of the first chamber.

2. A device as claimed in claim 1, **characterized in that** the two chambers are delimited respectively by two rods forming pistons provided each with seals, the device comprising motive means for controlling the displacement of the two rods, and a gasometer arranged in the thermostat-controlled enclosure.

3. A device as claimed in claim 1, **characterized in that** first chamber (14) is delimited by a rod (17) forming a piston provided with seals (J), and the second chamber consists of the inner volume of a gasometer (44) fitted with a mobile piston (48) and placed on top of the first chamber.

4. A device as claimed in claim 2, **characterized in that** the body is fixed inside thermostat-controlled enclosure (7), the two mobile elements (17, 18) are respectively secured to two hangers (12, 13) that can each be shifted parallel to the two pistons by driving respectively into rotation threaded rods (8, 10) and motive means (ME1, ME2) for rotating separately or synchronously the rods associated with each hanger.

5. A device as claimed in claim 3, **characterized in that** the body is fixed inside thermostat-controlled enclosure (7), at least one of the mobile elements (17, 18) is secured to a hanger (13) that can be shifted parallel to the mobile element by driving into rotation respectively threaded rods (10) and motive means (ME1, ME2) for rotating separately or synchronously the rods associated with the hanger.

6. A device as claimed in claim 3 or 5, **characterized in that** piston (48) of gasometer (44) slides in a cylinder and it is provided with a rod (49) connected to motive means suited to limit the pressure in the cylinder to a set value, so as to be able to measure at this pressure the volume of gas released by the expansion of the mixture.

7. A device as claimed in claims 4 or 5, comprising stepping type motors or synchronous motors.

8. A device as claimed in any one of the previous claims, comprising a control unit (CA) for controlling the motive means and the optical display assembly.

9. A device as claimed in the previous claim, **characterized in that** the control unit comprises a module (32) for controlling motive means (ME1, ME2) in order to drive threaded rods (8, 10) and an optical coding measuring circuit (33) for detecting the motions transmitted to the threaded rods and for converting them into volume variation measurements.

10. A device as claimed in claim 8, **characterized in that** control unit (CA) comprises a module (36) for controlling the illumination of first chamber (14) through first optical element (19), and for forming the image of an interface between phases through second optical element (20).

11. A device as claimed in any one of claims 7 to 10, comprising a vibrator (SU) for stirring the fluids in first chamber (14), control unit (CA) comprising a circuit (37) producing the signal to be applied to the vibrator.

12. A device as claimed in any one of claims 7 to 11, comprising a means (25-26) for measuring the pressure in first chamber (14), control unit (CA) comprising a circuit (38) for storing calibration factors of this measuring means (25-26) as a function of a set temperature value, associated with an element (39) for selecting the calibration factors to be chosen as a function of this set temperature value assigned, in order to make the response of measuring means (25-26) independent thereof.

13. A device as claimed in any one of the previous claims, comprising pressure measuring means including at least one membrane pressure detector (P1) arranged in a cavity provided in the wall of the first chamber, the membrane being flush with the inner surface of this chamber so as to limit clearance volumes.

14. A device as claimed in claim 2, comprising viscosity measuring means (27) connected between the first and the second chamber (14, 15).

15. A device as claimed in any one of the previous claims, comprising a microcomputer (43) programmed to perform automatic measurement procedures.
